Europäisches Patentamt

(19)  European Patent Office

Office européen des brevets

(11)  EP 0 697 217 A1

(12)  EUROPEAN PATENT APPLICATION

(43) Date of publication:
21.02.1996  Bulletin 1996/08

(51) Int. Cl.⁶: **A61L 15/60**

(21) Application number: 95111658.1

(22) Date of filing: 25.07.1995

(84) Designated Contracting States:
AT BE CH DE DK ES FR GB GR IE IT LI LU MC NL PT SE

(30) Priority: 01.08.1994 US 283559
02.06.1995 US 460623

(71) Applicant: PEARLSTEIN, Leonard
Gladwyne, PA 19035 (US)

(72) Inventors:
• Suskind, Stuart
Wayne, PA 19087 (US)
• Pearlstein, Leonard
Gladwyne, PA 19035 (US)

(74) Representative: Sama, Daniele
I-20129 Milano (IT)

(54)  **High performance super-absorbent material and absorbent devices containing the same**

(57)  A high performance absorbent particulate composition and a method of preparation in which a non-colloidal solid filler core is substantially encapsulated within a layer of hydrogel forming polymer is disclosed. Also disclosed are absorbent devices using the high performance absorbent particulate composition and methods of making these devices.

FIG. 5

Printed by Rank Xerox (UK) Business Services
2.9.9/3.4

## Description

## BACKGROUND OF THE INVENTION

The present invention relates to absorbent particles and the use of such particles in absorbent articles such as disposable diapers and sanitary pads.

Generally, absorbent articles are designed to have at least three distinct layers:

(1) a liquid pervious topsheet of bonded fibers usually referred to as a non-woven or an apertured film;
(2) an absorbent core containing mainly hydrophilic fibers such as a loose mat of pulp fibers usually referred to as "fluff" often contributing as much as 90% of the overall thickness of the article; and
(3) a liquid barrier outer film which is usually a polyolefin at a thickness of about 0.5-1.5 mils.

Fluids passing through the topsheet are distributed by the pulp fibers and are held within the interstices of the pulp web. Increases in pulp content generally lead to increases in the absorbent capacity of these devices. In light of the relatively low cost of pulp, absorbent articles are often designed with thick, bulky cores to provide high capacity in the absorbent article. The inherent disadvantages of such bulkiness are lack of comfort, visibility through clothing, and the inability of the article to conform to the shape of the body leading to unpredictable shifting and even fluid leakage. Importantly, these bulky items require precious storage space in distribution and in ultimate usage.

The development of superabsorbent polymers (SAP) which absorb at least 3-4 times as much fluid as pulp, has led to the successful design of much thinner products in which a substantial portion of the fluff has been replaced by SAP granules. While the consumer has accepted and even preferred these thinner articles, certain performance attributes are still not satisfactory and improvements are sought. For example, leakage is still one of the most important and the most critical deficiency remaining in disposable diaper design.

One approach taken to solve this problem has been to incorporate yet more SAP into the product. It is not uncommon in ultra-thin diapers to use as much as 6-15 grams of SAP granules per diaper accounting for up to 60% by weight of the absorbent core in order to achieve significant reductions in bulk or thickness and still provide needed performance.

SAP, generally, are polymeric materials containing water-insoluble long chain molecules with a low degree of cross-linking which are capable of forming hydrogel networks. In the presence of water or aqueous solutions such as body fluid, these hydrogel networks swell into a soft, resilient "jelly-like" material. When the swelling fluid is 0.9% saline, urine, or synthetic urine, these polymers may ultimately swell up to about 25-40 times their original weight. On the other hand, pulp fibers have a capacity to swell by a factor of only about 7-10 times by comparison.

The SAP materials are typically produced as granules which may then be mixed with pulp fibers during the formation of the absorbent core. Thus, with such highly absorbent granular material, it becomes possible to design and produce absorbent articles with roughly 1/2 to 1/3 of the bulkiness of the 100% pulp core. Reduction in volume of this nature is the subject of numerous U.S. Patents including for example, U.S. Patent Nos. 4,950,264 (Osborn); 4,467,012 (Pederson); and 4,217,901 (Bradstreet), all incorporated in their entirety by reference herein.

There is unfortunately a disadvantage associated with this improvement. For while SAP is about three-fold more absorbent than pulp, its cost may be about four-fold higher. It is not surprising, therefore, that considerable effort has been dedicated toward maximizing or optimizing the cost effectiveness of the superabsorbent. These efforts are the subject of numerous U.S. patents. For example, particle size, modules, degree of neutralization, and residual monomers, are discussed in U.S. Patent Nos. Re. 32,649 (Brandt) and 5,061,259 (Goldman et al.), both incorporated in their entirety by reference herein.

Furthermore, technical contributions were reported at the Advances in Superabsorbent Polymers Symposium, Fall Meeting 1993 of the American Chemical Society, as published in the Proceedings of the Division of Polymeric Materials: Science and Engineering (Masuda, p. 464; Nagorski, p. 560), incorporated herein by reference.

Prior to the trend toward thinner diapers, SAP was designed initially for maximum capacity and later with increased cross-linking for improved gel stability and absorbency under a load. With the trend toward thinner construction, the speed of liquid acquisition and distribution are also important properties. Accordingly, while SAP particles usually swell to capacity after about one hour exposure to fluid, the uptake rate during the first ten to twenty minutes is now considered critical.

It has been recognized that the rate of absorption could be increased through higher cross-link density of the SAP since the resulting increase in gel strength helps to maintain particle identity during swelling thus reducing particle coalescence. The effect of the latter phenomenon known as "gel blocking" is to block the open spaces in the web, causing a decrease in the rate of absorption. The undesirable drawback with increased cross-linking is the associated reduction in fluid capacity. In U.S. Patent Nos. 4,587,308 (Makita) and 4,507,438 (Obayashi) (both incorporated in their entirety by reference herein), particles are subjected to cross-linking on the particle surface thereby increasing surface gel strength without compromising the swell capacity within the particle.

2

In U.S. Patent No. 3,932,322 (Duchane) (incorporated in its entirety by reference herein), the tendency for particle agglomeration is reduced by admixing a small amount of very fine inorganic oxide particles which tend to coat the SAP particles.

The problem of reduced rate due to blocking of fluid is also addressed in U.S. Patent No. 5,147,343 (Kellenberger) (incorporated in its entirety by reference herein), wherein the size of the superabsorbent particle is selected to be larger than the pore size of the absorbent core (i.e., at least about 100 microns). This design feature is claimed to provide improved absorbency under a load or under the weight of the user's body.

The importance of particle size is further taught in U.S. Patent No. 5,180,622 (Berg et al.) (incorporated in its entirety by reference herein). This patent, more specifically, discloses that surface area of the particles controls the rate of fluid uptake. Since small particles have the more favorable ratio of surface area to mass, theory would predict a higher absorption rate with relatively small particles, i.e., about 50 ^mm. In fact, due to ease of packing, particles this size tend to form a mass of coagulated gel and fluid and flow is impeded by their "gel blocking." In the Berg et al. patent, the trade-off between fluid uptake and gel coagulation is resolved through a process which chemically links small particles into a larger cluster or agglomerate. These new particles have significantly higher swell rates than the precursor particles based on the high surface to mass ratio; however, this benefit is offset to some degree by increased processing cost. Furthermore, it is known that the efficiency of SAP in absorbing fluid content decreases as SAP content increases from, for example, about 20% by weight to about 60% by weight.

Another approach to solving the trade-off problem is to simply use larger quantities of larger particles; the drawback here is that increased volumes of SAP create additional expense for the manufacturer.

## SUMMARY OF INVENTION

In light of the high cost of superabsorbent polymers and the need to find efficient and improved ways to utilize their properties, it is desirable to provide granular superabsorbent polymers in a novel form which offers important improvements and advantages over previous compositions.

Improvements in cost and performance of disposable absorbent devices such as, for example, disposable baby diapers, garments and pads designed to aid in fluid control for adult incontinence, and sanitary napkins for feminine hygiene are needed where fluid control properties are achieved through selection of absorbent fibers such as pulp and superabsorbent polymers typically in the form of granules.

It is especially desirable to increase the ratio of superabsorbent polymer to absorbent fibers to levels as high as 50% and even 60% by weight superabsorbent to provide thin, compact, and low cost devices that are comfortable to the wearer, highly effective in absorbing fluids, and reasonable in cost. Since the cost of superabsorbent polymer is relatively high in comparison to pulp, a successful design of the absorbent products which provides a high level of value to the end-user will necessarily make the most effective use of the superabsorbent employed.

Accordingly, to achieve these and other advantages and in accordance with the purpose of the present invention, as embodied and broadly described herein, the present invention relates to a plurality of non-agglomerated and discrete absorbent particles having a non-colloidal, water-resistant solid core and a hydrogel forming polymer substantially encapsulating the solid core.

An additional feature of the present invention is a method of making these absorbent particles having a particle size of $D_{AP}$. In this method, non-colloidal water resistant solid particles having a particle size $D_{CP}$ are individually suspended in a water immiscible solvent in the presence of a surface active agent. An ethylenically unsaturated monomer capable of polymerizing into a hydrogel forming polymer is suspended in the water immiscible solvent along with an initiator. The monomer is then polymerized such that the non-colloidal, water resistant solid particles are individually and substantially encapsulated with a polymer coating thickness T by the hydrogel forming polymer to form an absorbent particle having a particle size $D_{AP}$. In this method of making the plurality of absorbent particles, $D_{AP}$ has a value in the range of about 30 microns to about 2000 microns, $D_{CP}$ has a value in the range of about 10 microns to about 150 microns, and T has a value in the range of about 10 microns to about 995 microns.

An additional feature of the present invention is an absorbent device having fibers and a plurality of absorbent particles of the present invention. The absorbent device can be, for example, a disposable diaper, a female sanitary napkin, a urinary incontinence pad for adults, a bed pad, a pad for absorbing fluids from food, or a bandage.

The present invention also relates to a process for manufacturing absorbent devices of the present invention wherein absorbent particles of the present invention are interdispersed into an absorbent core comprising fibers. This absorbent core containing the absorbent particles is then interposed between a liquid pervious topsheet and a liquid impervious backsheet. The edges of the topsheet and backsheet are then sealed together by techniques well known in the art to create a liquid barrier around the entire circumference of the absorbent device.

Additional features and advantages of the present invention will be set forth in part in the description which follows, and in part will be apparent from the description, or may be learned by practice of the present invention. The objectives and advantages of the invention will be realized and attained by means of the elements, combinations, composition, and process particularly pointed out in the written description and appended claims, as well as the appended drawings and photographs.

To achieve additional objectives in accordance with the purpose of the present invention as embodied and broadly described herein, the present invention relates to an absorbent device comprising a combination of hydrophilic and/or hydrophobic fibers and a plurality of superabsorbent polymer particles wherein said particles comprise a non-colloidal, non-water-swellable solid core, which is substantially encapsulated by a hydrogel forming polymer.

The present invention further relates to a process for making these absorbent particles which includes the steps of individually suspending non-colloidal solid particles in a water immiscible solvent in the presence of a surface active agent and then suspending in the water immiscible solvent, an aqueous solution of an ethylenically unsaturated monomer capable of polymerization into a hydrogel forming material, and an initiator. In the next step of the process, the monomer is polymerized such that the non-colloidal solid particles are individually and substantially encapsulated by the hydrogel forming polymer to form absorbent particles. After this polymerization step, the formed absorbent particles are separated from the solvent and dried.

By providing absorbent particles having a non-colloidal water resistant solid core substantially encapsulated by a hydrogel forming polymer, the polymer is readily available and is so structured physically to provide a high ratio of surface area to mass, a high rate of fluid absorption, a high level of gel stability, and a high absorbent capacity under load, thereby providing improved fluid absorbing efficiency when used in diapers and other absorbent composites, articles, and devices.

It is to be understood that both the foregoing general description and the following detailed description are exemplary and explanatory and are intended to provide further explanation of the present invention as claimed.

The accompanying drawings and photographs which are included to provide a further understanding of the present invention and are incorporated in and constitute a part of this specification, illustrate various embodiments of the present invention and together with the description serve to explain the principles of the present invention.

## BRIEF DESCRIPTION OF DRAWINGS

Figure 1 is a diagrammatic representation of an absorbent core in accordance with the present invention.

Figures 2-4 are diagrammatic representations of absorbent cores in accordance with the present invention showing various volumetric zones.

Figures 5 and 6 are microphotographs of absorbent particles of the present invention.

Figure 7 is a diagrammatic representation of an absorbent device showing various zones.

## DETAILED DESCRIPTION

Reference will now be made in detail to the present invention and various embodiments thereof, examples of which are illustrated in the accompanying drawings.

With respect to the absorbent particle(s) of the present invention, the particle comprises a non-colloidal, water-resistant solid core substantially encapsulated by a hydrogel forming polymer.

The non-colloidal water resistant solid core serves, in part, as a filler in order to provide a low cost core which is eventually substantially encapsulated by a hydrogel forming polymer. Generally, the size of the solid core, as measured by standard mesh screens, can range from about 10 microns to about 1500 microns, preferably from about 25 microns to about 1000 microns, and more preferably from about 100 microns to about 600 microns. The core can be any shape, e.g., spherical, oval, polyhedral, irregular, and non-spherical.

The weight ratio of solid core to hydrogel forming polymer in the absorbent particle of the present invention is at least about 1:4 and as high as about 9:1, and preferably from about 2:3 to about 2:1.

Typically, each particle of solid core is individually, discretely, and completely contained (i.e., encapsulated) by the hydrogel forming polymer. Occasionally, two or more solid core particles are contained in a single discrete absorbent particle. In some instances, a particle of solid core may not be completely encapsulated, but in most instances, and preferably, the particle of solid core is substantially (e.g., at least about 80% of the surface area of the core is covered with hydrogel forming polymer, preferably at least about 90%, and more preferably at least about 95%), if not completely, encapsulated by the hydrogel forming polymer.

It is to be understood that the term "water resistant" is herein used for purposes of the present invention to mean that the non-colloidal solid core is insoluble in water and aqueous solutions and non-swellable (at temperatures below about 90° F.) in water and aqueous solutions yet it is wettable with water and aqueous solutions.

Although there is no intention to limit the present invention to any particular non-colloidal, water resistant solid core material, the solid core material can be, for example, one or more materials selected from any of the following groups as long as the core can be encapsulated by the hydrogel forming polymer:

a) water insoluble inorganic minerals including, but not limited to, silicon dioxide, titanium dioxide, magnesium oxide, antimony oxide; clay, talc, wollastonite; synthetic amorphous silica; calcium carbonate; hollow mineral spheres, and the like, including the minerals described in Van Nostrand's Scientific Encyclopedia (1989), Seventh Edition, pages 1864-65, incorporated herein by reference; and

b) water insoluble organic materials in particulate or granulated form including but not limited to cereals and cereal components such as hull, bran, flour, germ, and meal; nut shells, wood flour, sawdust, cellulose, microcrystalline cellulose, starch, gelatin, and the like.

Various mixtures and combinations of the foregoing materials can also be used as core filler materials in accordance with the present invention.

With regard to the hydrogel forming polymer, certain preferred water insoluble polymeric compositions useful in the present invention are listed below. The polymers set forth below and containing acid groups can be, as an option, partially or completely neutralized with alkali metal bases either as the monomer or the polymer or both. While the list below contains many of the preferred polymers which may be used in accordance with the present invention, the present invention is not limited to just these polymers and generally polymers traditionally understood as SAP by those skilled in the art can also be used:

a) polyacrylic acid, polymethacrylic acid, polymaleic acid, copolymers thereof, and alkali metal and ammonium salts thereof;

b) graft copolymers of starch and acrylic acid, starch and saponified acrylonitrile, starch and saponified ethyl acrylate, and acrylate-vinyl acetate copolymers saponified;

c) polyvinyl alcohol, polyvinylpyrrolidone, polyvinyl alkylether, polyethylene oxide, polyacrylamide, and copolymers thereof;

d) copolymers of maleic anhydride and alkyl vinylethers; and

e) saponified starch graft copolymers of acrylonitrile, acrylate esters, vinyl acetate, and starch graft copolymers of acrylic acid, methyacrylic acid, and maleic acid.

The above exemplary polymers can be used in their linear state, or optionally, cross-linked either during the polymerization or after the core is encapsulated. This cross-linking can be achieved by methods known to those skilled in the art, including the use of a cross-linking agent. This cross-linking can be initiated in the presence of radiation or a chemical free radical initiator.

Polyfunctional cross-linking agents useful in the present invention include epichlorohydrin and related halo epoxy compounds, diglycidyl ether compounds, diisocyanates, polyaldehydes, and polyfunctional amines and imines.

Polyfunctional ethylenically unsaturated cross-linking agents include N,N',-methylene bisacrylamide, trimethylolpropanetriacrylate, ethylene glycol bismethacrylate, polyethylene glycol bismethacrylate, and divinyl benzene. The use of additional polymer cross-linking agents to modify the properties of gel forming polymers is well known and described in U.S. Patent No. 4,783,510 as well as by Yin, Y., Polyelectrolyte Gels, Chapter 6, American Chemical Society, 1992, both incorporated herein by reference.

Hydrogel forming polymers and methods of preparation are known in the art and these polymers can be used in the present invention including the polymers in which divalent cations are used as cross-linking agents as set forth in U.S. Patent Nos. 4,507,438; 5,145,906; 5,196,456; 5,250,642; and 4,295,987, all of which are incorporated herein by reference.

The particles of the present invention generally conform to the shape of the core particle. For example, where the core material is silica, the resulting absorbent particle is irregular in shape having a combination of flat and rounded surfaces and having various degrees of sharp and rounded corners and edges.

In the present invention, average absorbent particle size and absorbent particle size distribution can be achieved by appropriate selection of the core particle size. Through knowledge of the specific gravity and weight ratio of both the core material and hydrogel forming polymer, the growth in size of the absorbent particle is predictable.

The core particles of the present invention are substantially encapsulated by the polymer in such a way that the increase in particle diameter is predictable and controllable. It has been an objective in the field of superabsorbent polymers to provide granules within a controlled and predetermined particle size range. The present invention provides for the first time, a practical, low cost method for achieving this goal. This novel and new property of controlled particle size is represented by the following mathematical expression:

$$D_{AP} = D_{CP} + 2T$$

wherein

$D_{AP}$ is the diameter of the absorbent particle containing an encapsulated core particle;

$D_{CP}$ is the diameter of the core particle; and

T is the thickness of the superabsorbent or hydrogel forming polymer coating which encapsulates the core particle.

Within the scope of the present invention, $D_{AP}$ has a value in the range of about 30 microns to about 2000 microns. $D_{CP}$ has a value in the range of about 10 microns to about 150 microns. T has a value in the range of about 10 microns to about 995 microns.

The physical structure of these new particles is new and unique and imparts the desirable properties achieved through the present invention. These include:

1. The absorbent particles are separate and individual and predictable in size by means of the present invention.

2. The great majority of absorbent particles contain one core particle. A minor quantity of absorbent particles contain zero or two core particles.

3. The core particle is centrally located within the absorbent particle.

4. The rigidity and water resistance of the core particle provide an absorbent particle which is high in modulus and gel strength, contributing favorably to the absorbency under load.

5. The particles provide a high ratio of surface area to volume.

For example, an increase of about 50% in the ratio of polymer surface area to polymer volume is realized when comparing a SAP spherical particle of 100% polymer to an example of the current invention in which a spherical particle contains a spherical silica core of 200 ^m diameter with a 1:1 weight ratio of SAP to silica.

The coating thickness of the hydrogel polymer that encapsulates the filler core is preferably from about 2 ^m to about 3000 ^m, more preferably about 50 ^m to about 1000 ^m, and most preferably about 100 ^m to about 300 ^m.

The preferred number average molecular weight of the hydrogel forming polymer that is un-crosslinked (e.g., linear) is at least about 250,000, more preferably from about 250,000 to about 450,000, more preferably from about 256,000 to about 400,000.

Preferably the outer surface of the hydrogel forming polymer that encapsulates the solid core is cross-linked. This cross-linked surface creates a high cross-linked density shell. Accordingly, the particles of the present invention preferably have this shell and the hydrogel forming polymer beneath this shell is either un-crosslinked or cross-linked to a lesser degree than the shell. This shell prevents gel-blocking (i.e., absorbent particles sticking together during swelling) and also provides increased absorbency under a load.

It is also possible to have a cross-linking gradient wherein the outermost regions (including the outer surface) is more crosslinked than the inner regions of the hydrogel forming polymer. Also, the entire hydrogel forming polymer can be crosslinked, wherein the crosslink density is substantially the same or varies throughout the polymer coating.

The high crosslinked density shell preferably comprises from about 1% to about 50% of the overall thickness of the hydrogel forming polymer encapsulating the filler core and more preferably from about 1% to about 20%.

The superabsorbent coating which encapsulates the core particle may be further characterized physically as:

a) strongly adherent to the core surface; moreover, in those instances where the solid core surface does not adequately attract and hold the polymer coating, it may be desirable to add coupling agents to the system either in the form of an added ingredient or a comonomer in the polymer backbone;

b) continuous and without significant cracks, holes, or breaks; and

c) tough and abrasion resistant.

The attributes stated above are based upon examination of the absorbent particles of this invention under both optical and scanning electron microscopy. In addition, selected test procedures were conducted to measure the strength, cohesiveness, and adhesion of the coating to the core particle. These are described in Example 32.

While the process disclosed in the present invention may be conducted with surfactants (i.e., surface active agents) having an HLB factor of from about 2 to about 12; it is preferred that the range of HLB factor be from about 3 to about 7 and most preferably from about 3 to about 5. A preferred surface active agent or surfactant is SPAN 60® which is a fatty ester of a sugar. These types of surfactants are preferred because they minimize interparticle agglomeration. These findings are summarized in Table I, wherein particles of the present invention were prepared in the manner set forth in Example 1 except as noted in the Table and the particles contained 50 wt% silica and the polymer was not crosslinked. Optionally, surfactants that are fatty esters of a sugar that have been reacted with various levels of ethylene oxide can

be used.

## Table I

### PARTICLE SIZE DEPENDENCE

| Starting Sand Size (Mesh) | Surfactant Type | Surfactant Level | Net HLB | Final Particle Size Analysis | | | | | |
|---|---|---|---|---|---|---|---|---|---|
| | | | | On 40 >420 | 40-60 420-250 | 60-100 250-150 | 100-200 150-75 | Thru 200 <75 | (mesh size) (particle size)* |
| 60-100 | Tween 81 Span 60 | 1.1 phr** 1.1 phr | 7.4 | 89.10 | 9.00 | 1.80 | 0.15 | 0.00 | |
| 100-140 | Tween 81 Span 60 | 1.1 phr 1.1 phr | 7.4 | 89.00 | 6.40 | 2.35 | 2.10 | 0.14 | |
| 100-140 | Tween 81 Span 60 | 2.75 phr 2.75 phr | 7.4 | 92.05 | 4.84 | 2.36 | 0.62 | 0.13 | |
| 60-100 | Tween 81 Span 60 | 2.75 phr 2.75 phr | 7.4 | 85.86 | 7.35 | 3.64 | 2.86 | 0.72 | |
| F-75 as is | Span 60 | 2.2 phr | 4.8 | 25.61 | 58.48 | 14.35 | 1.47 | 0.10 | |
| F-75 as is | Tween 81 | 2.2 phr | 10.0 | 50.94 | 37.77 | 8.19 | 3.07 | 0.04 | |

```
    *   in microns
    **  phr = parts per hundred resin (polymer)
```

Various stabilizers, dispersants, dyes, pigments, diluents and the like, as well as anti-microbial agents, odor absorbing compounds, and perfumes can also be part of the absorbent particle of the present invention.

With regard to methods of making the absorbent particle of the present invention, the following steps, e.g., can be used:

a) individually suspending non-colloidal water resistant solid particles in a water immiscible solvent in the presence of a surface active agent (i.e., surfactant);

b) suspending in the water immiscible solvent an aqueous solution of an ethylenically unsaturated monomer capable of polymerization into a hydrogel forming material and an initiator;

c) polymerizing the monomer such that the non-colloidal water resistant solid particles are individually and substantially encapsulated by the hydrogel forming polymer to form absorbent particles; and

d) separating and drying the absorbent particles.

The hydrogel forming polymer can be crosslinked with polyfunctional cross-linking agents which are included in the aqueous solution in step (b) above. Alternately, polyfunctional cross-linking agents can be added at the end of step (c) and prior to step (d) above. Cross-linking can occur by suspending the absorbent particles in step (d) in a solvent containing a polyfunctional cross-linking agent.

In one preferred aspect of the present invention, the absorbent particle is prepared from partially neutralized acrylate-acrylic acid mixture dissolved in water which is added, along with potassium persulfate (a free radical initiator) to a suspension of silica particles (Ottawa Foundry Sand, Grade F-75, U.S. Silica Company) and surfactant in an organic solvent such as cyclohexane. This particular grade of sand is supplied with a particle size range of from about 50 to about 600 microns with about 85% by weight of the particles in the range of from about 100 to about 200 microns. Neutralization may be carried out with alkali metal hydroxides (e.g., sodium) or ammonium hydroxides.

Sufficient agitation of the suspension, e.g., with a stirrer, is provided to allow the polymerization mixture to adsorb onto the exterior surface of the core material, e.g., silica particles. The mixture is heated at a temperature of from about 40°C to about 60°C for about 2-3 hours to polymerize the sodium acrylate-acrylic acid mixture.

After polymerization is complete, the suspended particles are separated (e.g., by decantation of the liquid components), dried, lightly ground and then classified, e.g., by using a sieve. Prior to separation, azeotropic distillation can be used to remove water from the polymer of the absorbent particles formed.

The average particle size of the resulting absorbent particles of the present invention are preferably in the range of from about 30 microns to about 2000 microns, preferably from about 100 microns to about 600 microns.

Typically from about 90 to about 95% weight yield of absorbent particles are in the size range of from about 100 to about 600 microns. Microscopic examination of the individual particles in the presence of water reveals sand particles substantially or fully encapsulated by hydrogel forming polymers.

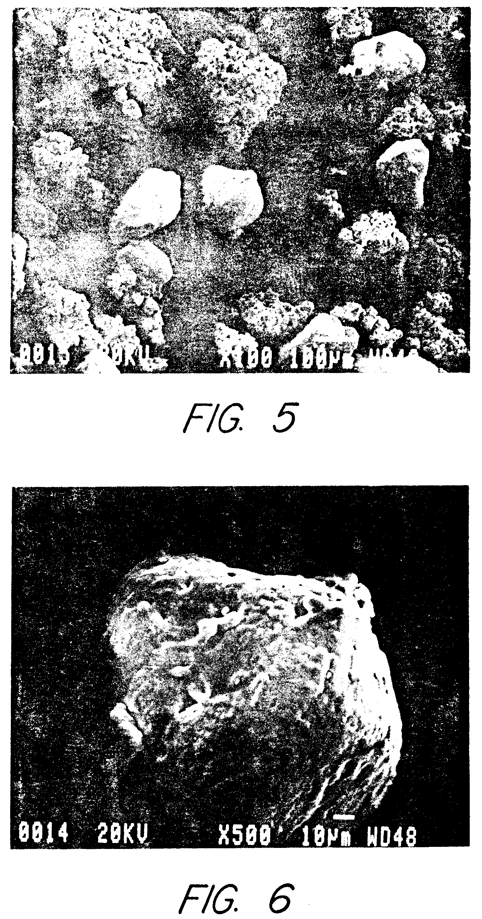

Figure 5 is a scanning electron micrograph at 100X magnification of an absorbent particle of the present invention in which silica particles (Grade F-75 Ottawa Sand) are coated with a superabsorbent polymer by a method similar to Example 9. Figure 6 is a 500X magnification of a particle in Figure 5. The various non-porous surface textures from rough to smooth is believed to be due to the degree of particle coalescence as the polymerizing droplets are adsorbed on the inner core.

Depending upon the properties desired in the absorbent particles, the process may be modified by one skilled in the art without undue experimentation with respect to:

a) Particle size distribution of the filler core.
b) Weight ratio of filler core to polymer.
c) Composition of the polymer and crosslink density.
d) Nature and quantity of surfactant.
e) Degree of agitation.
f) Polymerization initiator.

Alternate methods for the preparation of the particles of the present invention are equally useful in that individualization of the non-colloidal solid core particle with subsequent coating by a hydrogel forming polymer is possible. Examples include (1) a fluidized bed of particles in a vapor stream of monomer and initiator under polymerization conditions; and (2) mixing of the non-colloidal particle with monomer and initiator followed by atomization into a chamber which provides the necessary polymerization conditions. It is to be understood that the monomer and initiator are dissolved in a solvent such as water which is subsequently removed upon atomization.

Most superabsorbent polymers of commercial importance are prepared by the free radical induced polymerization of acrylic acid and salts thereof. In one process, an aqueous solution of partially neutralized monomer is polymerized in bulk followed by removal of water from the polymer mass and then grinding to the desired particle size. Particles with diameters below about 100 microns are generally considered to be an undesirable dust. In an alternate method, often referred to as "inverted suspension polymerization," the aqueous monomer solution, partially neutralized, is suspended as tiny droplets in a water immiscible solvent such as cyolohexane. After completion of the polymerization, water is removed and the polymer is recovered as specially shaped granules.

The above mentioned suspension process is described in U.S. Patent Nos. 4,340,706 and 4,418,163, both of which are incorporated herein in their entirety by reference. Additionally, this preparation method is described in Chapter 2 of Superabsorbent Polymers, ACS Symposium Series 573 (1994), and is also incorporated herein in its entirety by reference.

In order to more clearly define the process of the present invention the following is presented as descriptive of the present invention, but does not necessarily represent a limitation of the present invention:

a) Before the addition of monomer, a quantity of non-colloidal water resistant particles is suspended in the water immiscible solvent with the aid of a surfactant.
b) An aqueous solution of monomer and a water soluble free radical initiator are co-suspended.
c) The polymerizing suspended droplets of monomer are adsorbed at the surface of the non-colloidal water resistant core particle and subsequently coalesce into a coating which substantially encapsulates the core particle.
d) The attraction for and adsorption on the core particle by the monomer droplets during the polymerization, preferably starts with the early stages of polymerization.

Generally, an absorbent device of the present invention will have a topsheet, a backsheet, and a core interposed between the topsheet and the backsheet. The backsheet will typically be a liquid impervious type of backsheet, and the topsheet will be a liquid pervious type topsheet of nonwoven material or a liquid pervious or impervious material having apertures. The absorbent core can be any design known to those in the art, including the absorbent core designs used in association with superabsorbent polymers such as in the patents described below, which are all incorporated in their entirety by reference herein.

The absorbent core used in an absorbent device will generally comprise fibers and a plurality of absorbent particles. These absorbent particles of the present invention can exist as a separate layer in the absorbent core or be interdispersed with the fibers in any manner known to those skilled in the art. Furthermore, the absorbent particles of the present invention can be substituted for a portion or all of the superabsorbent polymers described in the patents below. The fibers used in the absorbent device of the present invention will preferably be hydrophilic in nature and be in the form of a web. The web is preferably collected on a forming screen which is a process known to those skilled in the art. The absorbent device will generally contain absorbent particles of the present invention wherein the non-colloidal solid core comprises about 20% to about 70% by weight and the solid core has a particle size of from about 10 microns to about 1500 microns.

The absorbent cores used in the absorbent devices of the present invention, including fibers and the manner in which the absorbent core is used with SAP as described in U.S. Patent Nos. 5,019,063; 5,300,358; 5,074,856; 4,944,735; 4,634,440; 4,342,314; 4,324,247; 4,217,901; 4,950,264; 5,147,343; 4,655,757; 5,061,259; and 5,286,770, can also be used with the absorbent particles of the present invention. A portion or all of the SAP referred to in these patents can be replaced with absorbent particles of the present invention.

Fibers used herein include, but are not limited to, mechanical pulp fibers, chemically-modified thermo-mechanical pulp fibers, cellulosic fibrous material, textile fibers, and the like. The absorbent can also be cellulose fluff, wood fluff, rayon, cotton, or meltblown polymers such as polyester, polypropylene, or coform.

The present invention also relates to a process for manufacturing absorbent devices of the present invention wherein absorbent particles of the present invention are interdispersed into an absorbent core comprising fibers. This absorbent core containing the absorbent particles is then interposed between a liquid pervious topsheet and a liquid impervious backsheet. The edges of the topsheet and backsheet are then sealed together to create a liquid barrier around the entire circumference of the absorbent device.

The high performance superabsorbent particles of the present invention will be further clarified by the following examples, which are intended to be purely exemplary of the present invention.

## Examples

The absorbent particles of the present invention were compared to a) a superabsorbent particle identified as FAVOR 800 which is commercially available from Stockhausen (Greensboro, N.C.). FAVOR 800 is believed to be a crosslinked polymer of partially neutralized acrylic acid; and b) a superabsorbent particle identified as ASAP 1000 which is commercially available from Chemdal (Palatine, Ill.). ASAP 1000 is also believed to be a crosslinked polymer of partially neutralized acrylic acid.

## Example 1

A 1000 ml round bottom flask equipped with nitrogen inlet and outlet tubes, a reflux condenser, a Dean Stark trap, a mechanical stirrer, a thermometer, and an additional funnel were purged with nitrogen. 400 ml of cyclohexane, 548 mg of polyoxyethylene 5 sorbitan monooleate (available as TWEEN 81® from ICI Americas, Inc., Wilmington, DE) and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the above surfactants were solubilized and the contents of the flask were allowed to cool to room temperature (i.e., approximately 25°C).

Concurrently with the foregoing, 40 ml acrylic acid and 10 ml deionized distilled water were stirred in a 250 ml round bottom flask under a nitrogen atmosphere with cooling provided by an ice bath. While cooling, 16.72 grams of sodium hydroxide dissolved in 50 ml deionized distilled water were added dropwise to the acrylic acid solution. 64 mg of potassium persulfate were then added to the partially neutralized acrylic acid solution. The mixture was stirred for approximately 10 minutes with cooling from the ice bath, then at ambient temperature to complete dissolution of the potassium persulfate and to form a sodium acrylate solution.

40 grams of Ottawa fine foundry sand (Grade F-75) with an AFS grain size of 75 were added to the cyclohexane/surfactant solution. The sodium acrylate solution was then added with vigorous stirring. The temperature of the reaction mixture was brought to 43°C and held there for 2½ hours. Water was removed as an azeotrope with cyclohexane and the solid dried to give 92.6 grams of off-white granular product.

60 grams of the above product, previously screened through 30 mesh onto 60 mesh, were slurried in 84 ml of methanol. 8.2 ml of 6.8 mg/ml aqueous solution of ethylene glycol diglycidyl ether (56.2 mg) were added. The reaction mixture was heated and stirred at 50-55°C for 2 hours. The solvent was removed under reduced pressure and the solid was oven dried at 50°C to give 62.3 grams of granular product of the present invention containing 44 wt.% silica.

Example 2

A 1000 ml round bottom flask equipped with nitrogen inlet and outlet tubes, a reflux condenser, a Dean Stark trap, a mechanical stirrer, a thermometer, and an addition funnel were purged with nitrogen. 400 ml cyclohexane, 548 mg of polyoxyethylene 5 sorbitan monooleate (available as TWEEN 81® from ICI Americas, Inc., Wilmington, DE) and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the contents of the flask were allowed to cool to room temperature.

Meanwhile, 40 ml of acrylic acid and 10 ml of de-ionized distilled water were stirred in a 250 ml round bottom flask under a nitrogen atmosphere with cooling provided by an ice bath. While cooling, 16.72 grams of sodium hydroxide dissolved in 50 ml de-ionized distilled water were added dropwise to the acrylic acid solution. 64 mg of potassium persulfate were added to the partially neutralized acrylic acid solution. The mixture was stirred for approximately 10 minutes with cooling from the ice bath, then at ambient temperature to complete dissolution of the potassium persulfate and to form a sodium acrylate solution.

62.58 grams of Ottawa fine foundry sand (Grade F-75) with an AFS grain size of 75 were added to the cyclohexane/surfactant solution. The sodium acrylate solution was then added with vigorous stirring. The temperature of the reaction mixture was brought to 43°C and held there for 2½ hours. Water and cyclohexane azeotrope were distilled off and the solid was dried to give 114.3 grams of off white, granular product.

Example 3 - Cross-Linked

36.5 grams, previously screened through 30 mesh onto 60 mesh, of the granular product of Example 2 was slurried in 35 ml of methanol. 4.0 ml of 6.8 mg/ml aqueous solution of ethylene glycol diglycidyl ether (27.44 mg) were added. The reaction mixture was heated and stirred at 50-55°C for 2 hours. The solvent was removed under reduced pressure and the solid was oven dried at 50°C to give 37.3 grams of granular product with 55 wt.% of silica.

Example 4

Example 4 was prepared in a manner similar to the procedure of Example 1. The core material was Hubercarb grade Q20-60 (J-W Huber Corporation, St. Louis, MO) comprised typically of 96.5% calcium carbonate (2% by weight of the size of calcium carbonate was 1170 microns-1560 microns), 2.0% magnesium carbonate, and 1.2% silica. The particle size distribution was characterized as 90% (by weight) greater than 200 microns and 10% (by weight) greater than 900 microns with a median particle size of about 450 microns. The weight ratio was 45 parts filler to 55 parts polymer. Example 4 was prepared without cross-linking agents.

Example 5

Example 5 was a repeat of Example 1 except that after removal of 44 gm of water as an azeotrope, 15.4 mg of ethylene glycol diglycidyl ether in 5 ml of methanol were added and the reaction mixture held for 3 hours at 50°C. At the end of this time, the remainder of the water was removed by azeotropic distillation (10 ml). The granular product was recovered and dried in an oven at 65°C for 4 hours.

Example 6

The objective of Example 6 was to increase the ratio of core filler to 72% by weight. Accordingly, a 1000 ml round bottom flask equipped with nitrogen inlet and outlet tubes, a reflux condenser, a Dean Stark trap, a mechanical stirrer, a thermometer, and an addition funnel were purged with nitrogen. 400 ml of cyclohexane, 548 mg of polyoxyethylene 5 sorbitan monooleate (available as TWEEN 81® from ICI Americas, Inc., Wilmington, DE) and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the contents of the flask were allowed to cool to room temperature.

Meanwhile, 20 ml acrylic acid and 10 ml deionized distilled water were stirred in a 250 ml round bottom flask under nitrogen atmosphere with cooling provided by an ice bath. While cooling, 8.36 grams of sodium hydroxide dissolved in 50 ml deionized distilled water were added dropwise to the acrylic acid solution. 33 mg potassium persulfate were added to the partially neutralized acrylic acid solution. The mixture was then stirred for approximately 10 minutes with cooling from the ice bath, then at ambient temperature to complete dissolution of the potassium persulfate and to form a sodium acrylate solution.

40 grams of Ottawa fine foundry sand (Grade F-75) with an AFS grain size of 75 were added to the cyclohexane/surfactant solution. The sodium acrylate solution was added with vigorous stirring. The temperature of the reaction mixture was brought to 55°C and held there for 2½ hours. 41 ml of water was removed as an azeotrope with cyclohexane. At that point, 15.4 mg of ethylene glycol diglycidyl ether in 4.8 ml of methanol was added and the reaction mixture held for 3 hours at 50°C. The reaction mixture was cooled to room temperature overnight.

The reaction was heated and an additional 12 ml water removed by azeotropic distillation. The reaction mixture contains some granular solid and some solid adhering to the wall of the flask. The granular product was recovered and dried in an oven at 50°C overnight. Deionized distilled water was added to the flask to swell off the solid adhering to the wall of the flask. The material was collected in a glass dish and allowed to dry. The solid exists as a mixture of small granules and larger ½-inch agglomerates.

Example 7

A 1000 ml round bottom flask equipped with nitrogen inlet and outlet tubes, a reflux condenser, a Dean Stark trap, a mechanical stirrer, a thermometer, and an addition funnel were purged with nitrogen. 400 ml of cyclohexane, 549 mg of polyoxyethylene 5 sorbitan monooleate (available as TWEEN 81® from ICI Americas, Inc., Wilmington, DE), and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the contents of the flask were allowed to cool to room temperature.

Meanwhile, 40 ml acrylic acid and 10 ml deionized distilled water were stirred in a 250 ml round bottom flask under nitrogen atmosphere with cooling provided by an ice bath. While cooling, 16.72 grams of sodium hydroxide dissolved in 50 ml deionized distilled water were added dropwise to the acrylic acid solution. 64 mg potassium persulfate were then added to the partially neutralized acrylic acid solution. The mixture was stirred for approximately 10 minutes with cooling from the ice bath, then at ambient temperature to complete dissolution of the potassium persulfate and to form a sodium acrylate solution.

27.5 gm of corn bran (Illinois Cereal Mills lot 99C-30) were added to the cyclohexane/surfactant solution. The sodium acrylate solution was then added with vigorous stirring. The temperature of the reaction mixture was brought to 55°C and held there for 2½ hours. 43 gms of water were removed as an azeotrope with cyclohexane. A particulate phase and a fused phase were both present at the end of this time. The solid phase was removed (approximately 35 gm of material). At that point, 4.3 ml of solution containing 8.5 mg ethylene glycol diglycidyl ether were added to the reaction mixture and the reaction mixture held for 3 hours at 50°C. At the end of this time, the remainder of the water was removed by azeotropic distillation (10 ml). The granular product was recovered and dried in an oven at 65°C for 4 hours.

Example 8

In Example 8, 4.0 gm of material prepared in the same manner as Example 6 were placed in a 50 ml flask along with 5.0 ml methanol plus 0.55 ml of a solution of ethylene glycol diglycidyl ether in methanol (10 mg/ml). The mixture was stirred at 55°C for 2 hours and then separated and dried.

Example 9

A 1000 ml round bottom flask equipped with nitrogen inlet and outlet tubes, a reflux condenser, a Dean Stark trap, a mechanical stirrer, a thermometer, and an addition funnel were purged with nitrogen. 400 ml of cyclohexane, 550 mg of polyoxyethylene 5 sorbitan monooleate (available as TWEEN 81® from ICI Americas, Inc., Wilmington, DE) and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the contents of the flask were allowed to cool to room temperature.

At the same time, 40 ml acrylic acid and 10 ml deionized distilled water were stirred in a 250 ml round bottom flask under nitrogen atmosphere with cooling provided by an ice bath. While cooling, 16.72 grams of sodium hydroxide dissolved in 50 ml deionized distilled water were added dropwise to the acrylic acid solution. 64 mg potassium persulfate were added to the partial neutralized acrylic acid solution. The mixture was stirred for approximately 10 minutes with cooling from the ice bath, then at ambient temperature to complete dissolution of the potassium persulfate to form a sodium acrylate solution.

51.2 grams of Ottawa fine foundry sand (Grade F-75) with an AFS grain size of 75 were added to the cyclohexane/surfactant solution. The sodium acrylate solution was then added with vigorous stirring. The temperature of the reaction mixture was brought to 54°C and held there for 2½ hours. Water was removed as an azeotrope with cyclohexane. 21 gms of water was removed as an azeotrope with cyclohexane. A particulate phase had formed at this point. At that point 3.4 ml of a methanol solution containing 10.2 mg ethylene glycol diglycidyl ether were added and the reaction mixture held for 2 hours at 50°C. Next, 23 ml of water was removed by azeotropic distillation. At that point another 3.4 ml of a methanol solution containing 10.2 mg ethylene glycol diglycidyl ether were added and the reaction mixture held for 2 more hours at 50°C. At the end of this time, the remainder of the water was removed by azeotropic distillation (14 ml). The granular product was recovered and dried in an oven at 65°C for 4 hours. The total yield was 103.2 gm.

Example 10

A 1000 ml round bottom flask equipped with nitrogen inlet and outlet tubes, a reflux condenser, a Dean Stark trap, a mechanical stirrer, a thermometer, and an addition funnel were purged with nitrogen. 400 ml of cyclohexane, 550 mg of polyoxyethylene 5 sorbitan monooleate (available as TWEEN 81™ from ICI Americas, Inc., Wilmington, DE) and 540 mg of sorbitan monostearate (available as SPAN 60® from ICI Americas, Inc., Wilmington, DE) were added to the flask and the internal temperature brought to 42°C with stirring to disperse the sorbitan monostearate. After approximately 10 minutes, the contents of the flask were allowed to cool to room temperature.

At the same time, 40 ml acrylic acid and 10 ml deionized distilled water were stirred in a 250 ml round bottom flask under nitrogen atmosphere and with cooling provided by an ice bath. While cooling, 16.72 grams of sodium hydroxide dissolved in 50 ml deionized distilled water were added dropwise to the acrylic acid solution. 64 mg potassium persulfate were added to the partially neutralized acrylic acid solution. The mixture was stirred for approximately 10 minutes with cooling from the ice bath, then stirred at ambient temperature to complete dissolution of the potassium persulfate and form a sodium acrylate solution.

30 gms of corn starch (Argo brand) were added to the cyclohexane/surfactant solution. The sodium acrylate solution was added with vigorous stirring. The temperature of the reaction mixture was brought to 55°C and held there for 2½ hours. 42 gms of water were removed as an azeotrope with cyclohexane. A particulate phase has formed at this point. At that point 5.2 ml of a methanol solution containing 15.4 mg ethylene glycol diglycidyl ether were added and the reaction mixture held for 3 hours at 50°C. At the end of this time, the remainder of the water was removed by azeotropic distillation (16 ml). The granular product was recovered and dried in an oven at 65°C for 4 hours. The total yield was 82.9 gm.

Example 11

In Example 11, 4.0 gm of material prepared in the same manner as Example 1 were placed in a 50 ml flask along with 5.0 ml methanol plus 0.55 ml of a solution of ethylene glycol diglycidyl ether in methanol (10 mg/ml). The mixture was stirred at 55°C for 2 hours and then separated and dried.

Test Procedures

1. Swell Time to Gel

A sample is passed through U.S. 30 Screen and collected on U.S. 60 mesh. A test specimen, 0.25 gm, is placed in a 7.0 ml vial. Add 2.5 gm of 1.0% sodium chloride. Observe and record time for meniscus to disappear as sample gels.

2. Capacity

Place 0.3 gm on aluminum pan and weigh. Add 10 ml of 1% saline solution; cover with film and let stand for 1½ hours. Discard excess solution and weigh. Calculate:

$$\frac{(\text{wt. of absorbent particle} + \text{saline}) - \text{wt. of absorbent particle}}{\text{wt. of absorbent particle}}$$

3. Absorbency Under an External Load

Purpose

Determine the amount of fluid absorbed by absorbent materials while placed under an external load as a function of time.

Conditions

A 0.016 gram sample of absorbent material is placed on a 20.28 cm² area, and a pressure of 21,000 dynes/cm² (approximately 0.3 lb/in²) is applied.

Equipment

Electronic Balance (range: minimum of 200 g; accuracy: at least 0.001 g)
2.54 cm (1 inch) inner diameter (I.D.) cylinder (material: Plexiglass®) with a stainless steel screen (mesh: 100) fused to the cylinder bottom.
2.5273 cm (0.995 inch) piston (material: Plexiglass®) weighing 4.4 g.
100 g weight (balance calibration weight).
Timer

Controlled Atmosphere:   Temperature 23°C (73.4°F) Relative Humidity 50%

Procedure

1. Prepare 1 kg of 0.9% (w/w) NaCl solution or synthetic urine.
2. Screen absorbent material to a 30/60 cut and place the 30/60 cut of material in sealed storage containers to maintain constant product conditions.
3. Place 0.160 g of absorbent material onto pre-tared weighing paper. Record the actual absorbent material weight, $W_f$, on a record sheet.
4. Slowly add the absorbent material into the cylinder with the screened bottom.
5. Gently tap the cylinder until the material is evenly distributed on the mesh. If absorbent material is on cylinder wall, the test is re-run.
6. Place piston into cylinder.
7. Place 100 g weight on piston.
8. Weigh the combined cylinder, piston and test material. Record this weight, $W_c$, on the record sheet.
9. Pour thin layer of solution into pan.
10. Place filter paper into pan and allow the solution to saturate the filter paper.
11. Set timer for predetermined time interval (1 or 5 minutes) or a maximum time (i.e. 60 minutes). (Measurements conducted at various time intervals are used to calculate and develop an absorption curve for the absorbent material.)
12. Place the combined cylinder on the saturated filter paper and simultaneously start the timer.
13. Upon expiration of time, remove the combined cylinder and blot on paper toweling.
14. Weigh the combined cylinder. Record this weight $W_s t$, on the record sheet.
15. Repeat steps 11 through 14 until equilibrium is reached or a maximum test time is obtained. Expand the time interval as maximum absorbency is approached. A suggested interval sequence is 0, 1, 2, 5, 10, 30, 60, and 90 minutes.
16. Measure the displaced piston height from the bottom of the cylinder. Record this height H on the record sheet.
17. Upon completion of the absorbency test, remove the piston and observe the condition of the material. Record the observation on the record sheet.

Calculations:

$W_f$ Weight of the absorbent material
$W_c$ Dry weight of the combined cylinder
$W_s t$ Wet weight of the combined cylinder at time, t
$A_t$ Material absorbency under an external load at time, t
A Maximum material absorbency under load.
Absorbency under an external load

$$At = \frac{W_s t - W_c}{W_f}$$

$$A = (\max A^t)$$

The absorbency properties of representative examples of the present invention are shown below in Tables I and II.

Properties

In Example 1, F-75, a fine foundry sand, was covered with a preferred hydrogel forming polymer prepared from an aqueous solution of partially neutralized acrylic acid at a weight ratio of 45 parts filler core to 55 parts polymer.

Example 3 differed from Example 1 in weight ratio by using 55 parts filler core to 45 parts polymer. In Example 2, the polymer was not crosslinked and differed from Example 3 which was crosslinked.

Table II

| Absorbent Particle | Maximum Swell Capacity g saline/g | Swell Time to Gel seconds 10:1 |
|---|---|---|
| Favor 800 (Stockhausen) | 47 | 25 |
| ASAP 1000 (Chemdal) | 40 | 29 |
| Example Number | | |
| 1. | 27 | 14 |
| 2. | 50+ | 32 |
| 3. | 23 | 24 |
| 4. | 40+ | 120 |
| 5. | 70+ | 9 |
| 6. | 70+ | 44 |
| 7. | 70+ | 85 |
| 8. | 25 | 27 |
| 9. | 41 | 4 |
| 10. | 50 | 2 |
| 20. | 21 | >5 min. |
| 21. | 33 | >5 min. |
| 24. | 31 | >5 min. |
| 25. | 32 | >5 min. |
| 28. | 11 | >5 min. |
| 29. | 22 | >5 min. |
| 30. | 26 | >5 min. |
| 31. | 34 | 7 |

Table III

| Absorbency Under Load gm saline (1.0%) per gm | | | | | |
|---|---|---|---|---|---|
| | 1 minute | 5 minutes | 10 minutes | 20 minutes | 30 minutes |
| Example 1 | 11 | 13 | 14 | 15 | -- |
| Example 3 | 10 | 14 | 16 | 16 | 16 |
| Favor 800 | 7 | 11 | 15 | 20 | 23 |
| ASAP 1000 | 8 | 16 | 20 | 22 | -- |
| Example 8 | 6 | 11 | 11 | 12 | 12 |
| Example 9 | 5 | 5 | 6 | 6 | 7 |
| Example 10 | 5 | 6 | 6 | 7 | |
| Example 11 | 10 | 18 | 20 | 21 | 22 |
| Example 20 | 3.5 | 5.3 | 5.8 | 6.1 | 6.5 |
| Example 21 | 1.4 | 2.6 | 3.2 | 3.7 | 4.1 |
| Example 28 | 1.6 | 1.9 | 2.0 | 2.0 | 2.1 |
| Example 29 | 1.1 | 2.1 | 2.1 | 2.9 | 2.9 |
| Example 30 | 1.3 | 2.3 | 2.7 | 3.3 | 4.0 |
| Example 31 | 6.4 | 8.3 | 9.5 | 11.2 | 12.3 |

The highly competitive performance in rate and absorbency under a load of the product of the present invention as seen from the data in Tables II and III are unexpected and represent an important advance in the art.

The complete adsorption of the polymerizing droplets onto the surface of the filler core was surprising and unexpected. During the course of the preparation it appeared that the filler particles became encapsulated during the early stages of the polymerization. This suggests certain theoretical explanations for the complete adsorption mentioned above. For example, early in the initiation of the polymerization, the soft and perhaps "sticky" droplets are attached to the filler core surface by secondary chemical forces or bonding provided at least in part by the chemical nature of the filler core particle surface. Additionally, the core particle surface may provide a sufficiently high surface energy to be readily wetted by the polymerizing droplets. In general, those physical-chemical phenomenon that tend to effect or control the adsorption and heterogenous reactions of liquids on solid surfaces influence the desired formation of the encapsulated core particles.

Absorbent Cores of Fiber and Superabsorbent Particles Examples 12-16

Cellulose pulp fibers were mixed with superabsorbent particles in a laboratory machine capable of providing a suitable air stream and collection screen to collect the mixture in the form of a soft, fluffy web.

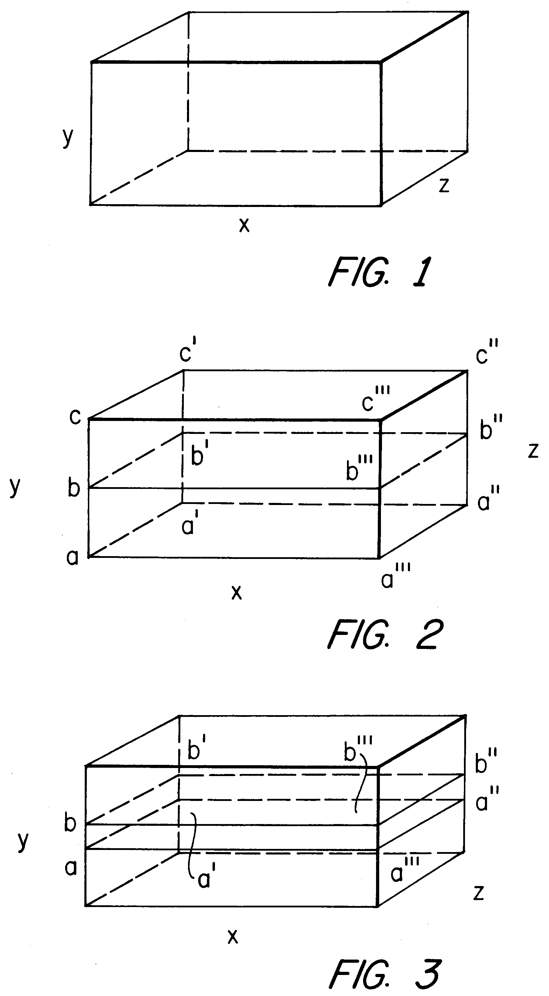

Figure 1 illustrates an absorbent core with a volume defined by the x, y, and z axis.

In Figure 2, two volumetric zones are defined, one by the boundaries ab, a'b', a"b", and a'"b'" and a second by bc, b'c', b"c", and b'"c'".

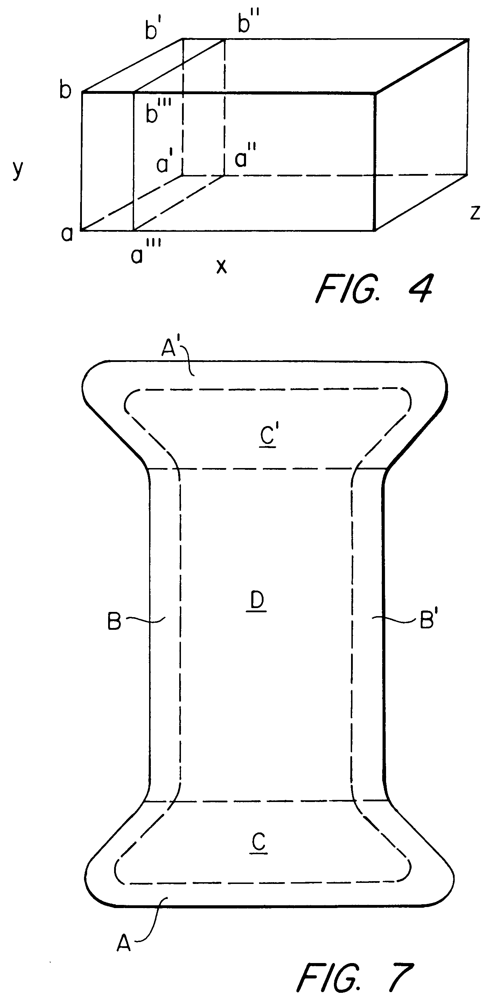

In Figures 3 and 4, the volumetric zone ab, a'b', a"b", and a'"b'" is defined.

The web of fiber and particles referred to herein as absorbent cores were approximately 9 inches in length, 4 inches in width, and after compression in a hydraulic press, 0.25 inch thickness. Referring to Figures 1 and 2, these dimensions are represented in an absorbent core by length "x," width "y," and thickness "z," respectively.

The absorbent cores of the present invention are defined by a series of planes, each of which is formed by the intersection of lines x and y moving in the z direction. These planes or any combination of multiples of planes provide volumetric zones (e.g., referring to Figure 3, showing a volumetric zone ab, a'b', a"b", a'"b'" and Figure 4 showing a zone ab, a'b', a"b", a'"b'") which are comprised of fiber, particles of the present invention, and air. These zones can occupy at least about 2% of the volume of an absorbent device in the dry state. Since the absorbent particles of the present invention are each individually comprised of a filler core and a superabsorbent polymer in the weight range of preferably about 30 to 70 to 70 to 30, it follows that the aforementioned volumetric zones are comprised of four components; namely, fiber, air, superabsorbent polymer, and filler. Furthermore, these components may be varied in both ratio and location in the core, thus providing a new degree of freedom in design of absorbent cores which may be optimized for both functionality and cost. The foregoing discussion on the particle composition and other variables associated with the particles of the present invention clearly shows the enormous magnitude of design options available with this new particle in combination with fiber and void space in the absorbent core.

Table IV lists the composition and properties of several examples of absorbent cores containing a) pulp fibers; and b) SAP and/or absorbent particles of the present invention.

Table IV

| Absorbent Core Properties | | | | | | | | | | |
|---|---|---|---|---|---|---|---|---|---|---|
| | | | | Time to Absorb 40 ml Saline, Successive Insults Seconds | | | | | | |
| Ex. | Absorbent Core Wt (gms) | Wt% SAP and/or Absorbent Particle | SAP and/or Particle Composition | 1 | 2 | 3 | 4 | Rewet (gm) | Capacity (gm) |
| 12 | 18.0 | 50 | ASAP-1000 | 8 | 21 | 55 | 90 | 7.6 | -- |
| 13 | 19.5 | 30 | ASAP-1000 | 6 | 11 | 27 | 40 | 8.5 | -- |
| 14 | 20.3 | 40 | Example 1 (4 pbw) ASAP-1000 (1 pbw) | 7 | 21 | 31 | 53 | 7.7 | |
| 15 | 18.8 | 40 | ASAP-1000 | -- | -- | -- | -- | -- | 363 |
| 16 | 19.1 | 40 | Example 1 (4 pbw) ASAP-1000 (1 pbw) | -- | -- | -- | -- | -- | 337 |

From the data in Table IV, it is shown that with the absorbent particles of the present invention, it is now possible to more closely optimize cost and performance in an absorbent device particularly with respect to performance attributes of capacity and rate.

More specifically, the data in Table III provides the basis for the design of absorbent devices with unique and heretofore unachievable properties without significant cost increases.

For example, in the device shown in Figure 7, a number of zones are indicated, each of which may have special importance due to location, in providing the desired functions of leakage prevention, providing a dry surface to the wearer, and reduction in bulk through replacement of pulp with SAP particles; and, importantly to achieve these at low cost.

The absorbent core of Example 14 contains 8.1 gm of SAP particles of which 1.6 gm are 100% superabsorbent polymer ASAP 1000. The remaining 6.5 gm of particles (Example 1) contain 56% SAP by weight or 3.6 gm SAP giving a total amount of SAP of 5.2 gm. In comparison with Example 12 which contains 9.0 gms of superabsorbent, the rate of absorption and surface dryness are significantly improved for the rate of absorption and roughly equivalent for surface dryness. These improvements are achieved at a significant reduction in raw material cost.

Absorbent devices such as that shown in Figure 7 may thus be optimized by the presently disclosed particles by employing, e.g., 75-100% more absorbent particles in, for example, zones A,A', B,B', and optionally C,C' thus achieving major improvements in leakage prevention through the presence of more available SAP surface area (i.e., a greater number of absorbent particles of the present invention can be made with the same amount of SAP used in present commercial absorbent devices).

Alternatively, an absorbent device of Figure 7 may be designed with high capacity particles as in Example 5 and located in the more inward zone D where control of large amounts of localized fluid is needed. As in the discussion above, such an improvement can be achieved without additional cost by using increased amounts of the lower cost particles of the present invention.

The absorbent particles of the present invention can be used in all types of absorbent devices where commercially available SAP particles are used. The absorbent particles of the present invention can fully or partially replace the commercially available SAP particles in absorbent devices such as those described in U.S. Patent Nos. 5,019,063; 5,300,358; 4,324,247; 4,342,314; 4,634,440; 5,074,856; and 4,944,735, all incorporated herein by reference in their entireties.

As shown in Table IV, for example, particles of the present invention can be blended with commercially available superabsorbent polymers to achieve a wide range of cost and performance. For instance, commercially available SAP particles can be combined with absorbent particles of the present invention in weight ratios of from about 1:9 to about 9:1. In addition, when the absorbent particles are used in an absorbent device such as a diaper or sanitary napkin, the hydrophilic fibers and absorbent particles are present in a weight ratio of from about 95:5 to about 2:3.

Techniques to increase the ratio of surface area to mass by forming aggregates of small SAP particles as taught, for example, in U.S. Patent No. 5,180,622 (Berg), or by incorporating blowing agents as for example in U.S. Patent No. 5,118,719 (both incorporated herein by reference) are applicable to the present invention as well with the added advantage of significantly reduced cost due to the filler core.

Example 17

In Examples 17 and 18, superabsorbent granules of the present invention were prepared by the general procedure of Example 1 with a silica content of 50% by weight.

To illustrate the highly cost effective performance of the present invention, female sanitary napkins were prepared according to the teachings of U.S. Patent No. 4,950,364, entitled "Thin, Flexible Sanitary Napkin" which is incorporated herein by reference. In particular, the present embodiment seeks to achieve a sanitary napkin which is thin and flexible and which is absorbent enough to absorb and contain medium to high menstrual flows.

Specifically, a laminate was prepared comprising a layer of airlaid bonded pulp sheet material having a thin film of pressure sensitive adhesive on one surface. The high performance superabsorbent granules of the present invention were uniformly sprinkled on the adhesive layer which had either a light (approximately ½ mil), medium (approximately 1 mil) or heavy (approximately 1½ mil) amount of adhesive, and then the sheet material was G folded into a laminate about 55 mm in width so that the superabsorbent granules were located within the inner folds of the laminate. Next the laminate was interposed between a fluid transfer layer of airlaid pulp sheet material and a polyethylene barrier film. A topsheet comprising a polyethylene film having three dimensional apertures was positioned on the outer face of the transfer layer airlaid material. The edges were sealed giving a sanitary napkin having a length of about 225 mm, a width of 70-100 mm, and a thickness of about 2.75 mm.

For comparative purposes, napkins were prepared with a commercial grade of superabsorbent (ASAP 1100, Chemdall) comprising granules of 100% superabsorbent polymer.

Table V below illustrates the absorbing properties of napkins containing the novel polymer sheath, absorbent particles of the present invention having only 50% by weight SAP and 50% by weight of silica.

## Table V

| Super Absorbent Polymer Granules | Adhesive; Application | Quantity, Granules Per Pad; GM | Absorbency Rate: Sec. | Rewet; GM | Stain; MM | Capacity; GM |
|---|---|---|---|---|---|---|
| Present | Fuller* 8130; Light | 0.80 | 2.80 | 2.20 | 78.00 | 44.00 |
| Invention | Fuller 8130; Light | 1.00 | 3.10 | 0.20 | 80.00 | 46.50 |
| | Fuller 8130; Light | 1.20 | 3.00 | 0.00 | 78.00 | 51.30 |
| | Fuller 8130; Med. | 0.80 | 3.10 | 0.00 | 75.00 | 41.70 |
| | Fuller 8130; Med. | 1.00 | 2.90 | 0.00 | 78.00 | 45.50 |
| | Fuller 8130; Med. | 1.20 | 3.00 | 0.00 | 80.00 | 53.00 |
| | Fuller 8130; Heavy | 0.80 | 3.20 | 0.19 | 75.00 | 42.30 |
| | Fuller 8130; Heavy | 1.00 | 3.00 | 0.00 | 80.00 | 44.90 |
| | Fuller 8130; Heavy | 1.20 | 3.00 | 0.00 | 80.00 | 49.20 |
| ASAP-1100 | Fuller 8130; Light | 0.80 | 2.90 | 0.00 | 78.00 | 42.80 |

| | | | | | | |
|---|---|---|---|---|---|---|
| | Fuller 8130; Light | 1.00 | 2.60 | 0.00 | 78.00 | 47.90 |
| | Fuller 8130; Light | 1.20 | 2.50 | 0.00 | 80.00 | 52.30 |
| | Fuller 8130; Med. | 0.80 | 2.70 | 0.00 | 75.00 | 43.40 |
| | Fuller 8130; Med. | 1.00 | 2.60 | | 75.00 | 50.20 |
| | Fuller 8130; Med. | 1.20 | 2.50 | 0.60 | 90.00 | 55.00 |
| | Fuller 8130; Heavy | 0.80 | 2.60 | 0.30 | 80.00 | 42.30 |
| | Fuller 8130; Heavy | 1.00 | 2.50 | | 75.00 | 50.20 |
| | Fuller 8130; Heavy | 1.20 | 2.70 | 0.00 | 75.00 | 52.40 |
| Present | NS 8423-23-4**; Light | 0.80 | 2.40 | 0.28 | 78.00 | 54.60 |
| Invention | NS 8423-23-4; Light | 1.00 | 2.30 | 0.00 | 72.00 | 57.90 |
| | NS 8423-23-4; Light | 1.20 | 2.30 | 0.00 | 72.00 | 62.90 |
| | NS 8423-23-4; Med. | 0.80 | 2.40 | 1.60 | 80.00 | 57.30 |
| | NS 8423-23-4; Med. | 1.00 | 2.60 | 0.00 | 72.00 | 58.40 |
| | NS 8423-23-4; Med. | 1.20 | 2.70 | 0.00 | 75.00 | 63.80 |
| | NS 8423-23-4; Heavy | 0.80 | 2.60 | 3.30 | 77.00 | 55.30 |
| | NS 8423-23-4; Heavy | 1.00 | 2.80 | 0.00 | 80.00 | 58.10 |
| | NS 8423-23-4; Heavy | 1.20 | 2.50 | 0.00 | 85.00 | 60.30 |
| ASAP-1100 | NS 8423-23-4; Light | 0.80 | 1.80 | 0.36 | 82.00 | 58.70 |
| | NS 8423-23-4; Light | 1.00 | 1.90 | 0.23 | 80.00 | 68.30 |
| | NS 8423-23-4; Light | 1.20 | 2.00 | 0.00 | 78.00 | 71.70 |
| | NS 8423-23-4; Med. | 0.80 | 2.40 | 1.60 | 80.00 | 57.30 |
| | NS 8423-23-4; Med. | 1.00 | 2.60 | 0.00 | 82.00 | 64.20 |
| | NS 8423-23-4; Med. | 1.20 | 2.20 | 0.00 | 78.00 | 70.00 |
| | NS 8423-23-4; Heavy | 0.80 | 2.50 | 0.00 | 85.00 | 57.50 |
| | NS 8423-23-4; Heavy | 1.00 | 2.40 | 0.00 | 78.00 | 55.60 |
| | NS 8423-23-4; Heavy | 1.20 | 2.20 | 0.34 | 78.00 | 70.20 |

*   H.B. Fuller Co. (St. Paul, MN)

**   National Starch & Chemical Corp. (Bridgewater, N.J.)

Example 18

In another embodiment of the present invention, a thick, fluffy absorbent pad comprising a combination of cellulose pulp fluff and superabsorbent granules were prepared and evaluated by laboratory fluid absorption tests.

The pads tested in this experiment were about 260 mm long, 60-70 mm wide, and 20 mm thick. The construction specifically comprised four layers of fluff pulp formed by "G" folding a "blanket layer" of pulp (7.8 gm) around an embossed and densified layer of pulp (4.1 gm). The pad further comprised a topsheet of thermally bonded polypropylene, a polyethylene fluid backsheet, and a resin bonded web of crimped PET stable fiber interposed between the topsheet and the fluff core. Superabsorbent granules were uniformly dispersed within the core through a salt shaker. In the case of the present invention, 1.4 gm of absorbent particles were distributed on the upper side of the embossed layer and 3.0 gm on the lower side. As a comparative example, Chemdall ASAP 1000 superabsorbent granules were similarly positioned, 1.0 gm being placed on the upper side and 3.0 gm on the lower side.

Absorption properties are summarized in Table VI.

Pads of this general construction are useful in feminine hygiene in the control of menstrual fluid as well as for the absorption and control of urine in light to moderate episodes of urinary incontinence.

TABLE VI

|  | Absorption Rate First 20 ml. Seconds | Absorption Rate Second 20 ml. Seconds | Rewet gm | Capacity gm |
|---|---|---|---|---|
| Present Invention | 1.00 | 2.30 | 1.20 | 235.00 |
| ASAP-1000 | 1.00 | 1.80 | 1.80 | 243.00 |

In Examples 17 and 18, absorbency rate, rewet, stain, and capacity were obtained as follows:

Absorbency Rate was determined as follows:

Apparatus: One 4" x 4" Lucite cylinder block with a 1" diameter opening in center of block; 1% dyed saline solution; stopwatch (to record elapsed time in seconds graduated to 0.1 second).

Procedure: Lay the sanitary product having a plastic film, absorbent core and cover sheet flat, so that it is free from wrinkles and folds, with the cover sheet up. Place the cylinder block in the center of the product with the cover sheet up. Measure 10 ml of 1% saline solution into the graduated cylinder. Pour 10 ml 1% saline solution from graduated cylinder onto product through opening in lucite block and immediately start the stopwatch. Allow the solution to flow onto the surface of the sanitary product. Stop the stopwatch as soon as the solution is completely absorbed. Record the absorption time for five specimens to the nearest 0.1 second. Determine the average absorption time for five specimens to the nearest 0.1 second. Average absorption time in seconds to the nearest 0.1 second and fluid volume.

Rewet was determined as follows:

Apparatus: One 4" x 4" Lucite cylinder block with a 1" diameter opening in center block; dyed 1% saline solution; VWR Filter paper, Grade #417, 9 cm in diameter or equivalent; flat plate weighing 0.05 kgs (4" x 4" x 1/8 Lucite square); 2.2 kilogram weight; 25 ml capacity cylinder; and top loading electronic balance, accurate to ± 0.01 g.

Procedure: Prepare five products for testing by placing flat on a level surface. Center the cylinder block on the coversheet of the product. Pour 10 ml of 1% saline solution into the cylinder block opening. Remove the cylinder block and allow the product to stand for 5 minutes. Weigh 10 filter papers and record weight. After 5 minutes, simultaneously place the weighed filter paper, clear Lucite plate, and 2.2 kilogram weight (approx. 0.5 psi) on the center of the product. Leave in this position for 15 seconds. Remove the weight and plate and weigh the filter papers. Determine the average rewet for five samples as follows: Final Filter Paper Weight (g) minus Initial Filter Paper Weight (g) equals Rewet (g).

Stain was determined as follows:

Apparatus: One 4" x 4" Lucite cylinder block with a 1" diameter opening in center of block; dyed (1%) saline solution; 10 ml graduated cylinder; stopwatch (accurate to ± 0.1 second); and steel ruler measuring 30 cm in length and graduated in 1 mm.

Procedure: Place cylinder block on the center of the coversheet, top side of the pad. Pour 10 ml of 1% saline solution into the cylinder block opening and allow the fluid to be absorbed. Remove the cylinder block as soon as absorption is complete. Allow the product to absorb the fluid. After two minutes, measure (in millimeters) the length of the stain from the furthest two points, keeping the ruler parallel to the length of the product. Average the readings from 5 samples and report in millimeters.

Total capacity was determined as follows:

Apparatus: Triple beam balance or top loading electronic balance (accurate to ± 0.1 gm); 0.9% saline solution; blotters (4" x 4" pulp board--ITT Rayonier PMXE); Whatman Filter paper, grade #1, or equivalent; Ohaus calibration weights of 1170.4 gm , ±0.4 gms (or equivalent); stopwatch; thermometer; Plexiglass® template measuring 4.75 x 14.0 cm; and nonwoven sample piece measuring 8" x 12".

Procedure: Record dry weight of sample piece without release tape. Wrap sample in nonwoven piece. Submerge sample piece in 0.9% saline (25°C) solution for 10 minutes. Drain for 2 minutes in a vertical position. Unwrap nonwoven from sample. Place 4" x 4" pulp square under bottom surface of saturated sample, poly side of product facing up. Place template with controlled weight onto product for 30 seconds. Continue above step until rewet value is less than 0.5 gm.

Record weight of wet sample piece.

$$\frac{\text{Wet Weight Sample - Release Tape}}{\text{Dry Weight Sample - Release Tape}} = \text{Total Capacity Value}$$

Example 19

A portion of the product from Example 5 remaining on a #30 mesh screen was ground lightly with a mortar and pestle and then combined with the portion that had passed through the screen. Using Nos. 30, 60, and 140 mesh screens, the particle size distribution of the combined material was determined and compared to the F-75 silica core material. This comparison shown in Table VII clearly illustrates the high degree of predictability of particle size offered by the present invention.

Table VII

|  | Silica F-75, Wt.% | Example 5, Wt.% |
|---|---|---|
| Less than 100 microns | 7 | 10 |
| Less than 150, greater than 100 microns | 22 | - |
| Greater than 150, less than 300 microns | 65 | - |
| Greater than 300 microns | 6 | - |
| Greater than 100, less than 260 microns | - | 26 |
| Greater than 260, less than 700 microns | - | 64 |

Example 20, CaCO$_3$ filler with surface crosslink

A 2000 ml round bottom flask equipped with nitrogen inlet and outlet tubes, a reflux condenser, a Dean Stark trap, a mechanical stirrer, a thermometer, and an addition funnel was purged with nitrogen. 800 ml cyclohexane, and 4.32 g Span 60 (HLB value 4.7) were added to the flask and the internal temperature brought to 42°C with stirring to disperse the Span 60. After approximately 10 minutes, the contents of the flask were allowed to cool to room temperature.

Meanwhile, 80 ml acrylic acid and 10 ml de-ionized distilled water were stirred in a 500 ml round bottom flask and cooled by an ice bath. While cooling, 33.5 grams of sodium hydroxide dissolved in 100 ml de-ionized distilled water were added dropwise to the acrylic acid solution. 128 mg potassium persulfate in 10 ml de-ionized distilled water were added to the partially neutralized acrylic acid solution. The mixture was stirred for approximately 10 minutes with cooling from the ice bath.

102.4 grams of calcium carbonate (Hubercarb Q 20 60) were added to the cyclohexane/surfactant solution. The sodium acrylate solution was added with vigorous stirring. The temperature of the reaction mixture was brought to 55°C and held there for 6 hours. The water and cyclohexane azeotrope were distilled over until 90 ml of water had collected. The mixture was allowed to cool overnight under nitrogen atmosphere.

The next morning, 1.34 g of ethylene glycol diglycidyl ether was added and the reaction mixture was heated to 55°C for 7 hours under nitrogen atmosphere, then allowed to cool overnight. The remaining water was removed with the water/cyclohexane azeotrope to give 190 g of off white granular material.

Example 21. Silica F-75 filler with internal crosslink

A 2000 ml round bottom flask equipped with nitrogen inlet and outlet tubes, a reflux condenser, a Dean Stark trap, a mechanical stirrer, a thermometer, and an addition funnel was purged with nitrogen. 800 ml cyclohexane, and 4.32 g Span 60 (HLB value 4.7) were added to the flask and the internal temperature brought to 42°C with stirring to disperse the Span 60. After approximately 10 minutes, the contents of the flask were allowed to cool to room temperature.

Meanwhile, 80 ml acrylic acid and 10 ml de-ionized distilled water were stirred in a 500 ml round bottom flask and cooled by an ice bath. While cooling, 33.5 grams of sodium hydroxide dissolved in 100 ml de-ionized distilled water were added dropwise to the acrylic acid solution. 96 mg of N,N'-methylene-bis-acrylamide were added to the aqueous solution and were stirred until dissolved. 128 mg potassium persulfate in 10 ml de-ionized distilled water were then added to the partially neutralized acrylic acid solution. The mixture stirred for approximately 10 minutes with cooling from the ice bath.

102.4 grams of silica (U.S. Silica, F-75) were added to the cyclohexane/surfactant solution. The sodium acrylate solution was added with vigorous stirring. The temperature of the reaction mixture was brought to 55°C and held there for 6 hours. The water and cyclohexane azeotrope were distilled over until 115 ml of water had collected. The solid product was collected and dried to give 191 g of white granular material.

Example 22. Silica F-75 filler control, no crosslinking

A 2000 ml round bottom flask equipped with nitrogen inlet and outlet tubes, a reflux condenser, a Dean Stark trap, a mechanical stirrer, a thermometer, and an addition funnel were purged with nitrogen. 800 ml cyclohexane and 4.32 g Span 60 (HLB value 4.7) were added to the flask and the internal temperature brought to 42°C with stirring to disperse the Span 60. After approximately 10 minutes, the contents of the flask were allowed to cool to room temperature.

Meanwhile 80 ml acrylic acid and 10 ml de-ionized distilled water were stirred in a 500 ml round bottom flask and cooled by an ice bath. While cooling, 33.5 grams of sodium hydroxide dissolved in 120 ml de-ionized distilled water were added dropwise to the acrylic acid solution. 128 mg potassium persulfate in 10 ml de-ionized distilled water were added to the partially neutralized acrylic acid solution. The mixture was stirred for approximately 10 minutes with cooling from the ice bath.

102.4 grams of silica (U.S. Silica, F-75) were added to the cyclohexane/surfactant solution. The sodium acrylate solution was added with vigorous stirring. The temperature of the reaction mixture was brought to 55°C and held there for 6 hours. The water and cyclohexane azeotrope were distilled over until 114 ml of water had collected. The solid product was collected and dried to give 195 g of white granular material.

Example 23. Molecular Weight Control

A 2000 ml three necked round bottom flask equipped with a mechanical stirrer, thermometer, Dean Stark apparatus, reflux condenser, and a nitrogen inlet was purged with nitrogen. 800 ml of cyclohexane and 4.32 g of Span 60 (ICI Americas, Inc., Wilmington, DE) were added to the flask and allowed to dissolve. After 10 minutes of stirring, the reaction mixture was homogeneous.

Meanwhile, 80 ml acrylic acid and 33.42 g of sodium hydroxide dissolved in 120 ml $H_2O$ were mixed together in an ice bath over a 5 minute period of time.

102.4 g of Ottawa fine foundry sand with an AFS grain size of 75 were added to the cyclohexane/surfactant solution. The sodium acrylate solution and 2.0 ^ml of 3-mercapto-1,2-propanediol (chain transfer agent for molecular weight control) were added with vigorous stirring. 128 mg of potassium persulfate were added and the temperature of the reaction mixture was brought to 50°C for 1 hour. The temperature was increased to 60.5°C for 5 hours. Water was removed as an azeotrope with cyclohexane and particles of the present invention were isolated. The polymer resulting from the chain transfer reaction had a number average molecular weight of 256,000, which was 60% smaller than the polymer isolated from an identical polymerization (?) that did not employ a chain transfer agent.

Example 24. Large Particle Size Silica Filler, on 60 mesh, >250 microns

A 2000 ml three necked round bottom flask equipped with a mechanical stirrer, thermometer, Dean Stark apparatus, reflux condenser, and a nitrogen inlet was purged with nitrogen. 800 ml of cyclohexane and 4.32 g of Span 60 (ICI Americas, Inc., Wilmington, DE) were added to the flask and allowed to dissolve. After 10 minutes of stirring, the reaction mixture was homogeneous.

Meanwhile, 80 ml acrylic acid and 33.42 g of sodium hydroxide dissolved in 120 ml $H_2O$ were mixed together in an ice bath over a 5 minute period of time.

102.4 g of larger than 60 mesh (250 microns and greater) Ottawa fine foundry sand with an AFS grain size of 75 were added to the cyclohexane/surfactant solution. The sodium acrylate solution was added with vigorous stirring. 128 mg of potassium persulfate were added and the temperature of the reaction mixture was brought to 50°C for 1 hour. The temperature was then increased to 60.5°C for 5 hours. Water was removed as an azeotrope with cyclohexane resulting in 181.3 g of particulate product.

Example 25. Small Particle size silica filler, 100-200 mesh, 75-150 microns

A 2000 ml round bottom flask equipped with nitrogen inlet and outlet tubes, a reflux condenser, a Dean Stark trap, a mechanical stirrer, a thermometer, and an addition funnel was purged with nitrogen. 800 ml cyclohexane and 4.32 g Span 60 (HLB value 4.7) were added to the flask and the internal temperature brought to 42°C with stirring to disperse the Span 60. After approximately 10 minutes, the contents of the flask were allowed to cool to room temperature.

Meanwhile, 80 ml acrylic acid and 20 ml de-ionized distilled water were stirred in a 500 ml round bottom flask and cooled by an ice bath. While cooling, 33.5 grams of sodium hydroxide dissolved in 100 ml de-ionized distilled water were added dropwise to the acrylic acid solution. 128 mg potassium persulfate in 10 ml de-ionized distilled water were added to the partially neutralized acrylic acid solution. The mixture stirred for approximately 10 minutes with cooling from the ice bath.

102.4 grams of silica (U.S. Silica, F-110, previously screened to 100-200 mesh) were added to the cyclohexane/surfactant solution. The sodium acrylate solution was added with vigorous stirring. The temperature of the reaction mixture was brought to 55°C and held there fob 6 hours. The water and cyclohexane azeotrope was distilled over until 115 ml of water had collected. The solid product was collected and dried to give 174 g of off-white granular material.

Example 26. Hardwood particles filler

A 2000 ml three necked round bottom flask equipped with a mechanical stirrer, thermometer, Dean Stark apparatus, reflux condenser, and a nitrogen inlet were purged with nitrogen. 800 ml of cyclohexane and 4.32 g of Span 60 (ICI Americas, Inc., Wilmington, DE) were added to the flask and allowed to dissolve. After 10 minutes of stirring, the reaction mixture was homogeneous.

Meanwhile, 80 ml acrylic acid and 33.42 g of sodium hydroxide dissolved in 120 ml $H_2O$ were mixed together in an ice bath over a 5 minute period of time.

50 g of 60 mesh hardwood flour (Composition Materials of America, Inc.) were added to the cyclohexane/surfactant solution. The sodium acrylate solution was added with vigorous stirring. 128 mg of potassium persulfate were added and the temperature of the reaction mixture was brought to 50°C for 1 hour. The temperature was then increased to 60.5°C for 5 hours. Water was removed as an azeotrope with cyclohexane resulting in 139.0 g of particulate product.

Example 27. Walnut shell particulate filler

A 2000 ml three necked round bottom flask equipped with a mechanical stirrer, thermometer, Dean Stark apparatus, reflux condenser, and a nitrogen inlet was purged with nitrogen. 800 ml of cyclohexane and 4.32 g of Span 60 (ICI Americas, Inc., Wilmington, DE) were added to the flask and allowed to dissolve. After 10 minutes of stirring, the reaction mixture was homogeneous.

Meanwhile, 80 ml acrylic acid and 33.42 g of sodium hydroxide dissolved in 120 ml $H_2O$ were mixed together in an ice bath over a 5 minute period of time.

50 g of 100 mesh Walnut Shell flour (Composition Materials of America, Inc.) were added to the cyclohexane/surfactant solution. The sodium acrylate solution was added with vigorous stirring. 128 mg of potassium persulfate were added and the temperature of the reaction mixture was brought to 50°C for 1 hour. The temperature was increased to 60.5°C for 5 hours. Water was removed as an azeotrope with cyclohexane resulting in 100.3 g of particles of the present invention.

Example 28. Surface Crosslinking by Calcium Ions

10.0 g of encapsulated particles from Example 22 and 30 ml of cyclohexane were added to 100 ml round bottom flask equipped with a magnetic stir bar, Dean Stark apparatus, and reflux condenser. 1.8 g of calcium chloride dissolved in 5 ml of $H_2O$ were then added with vigorous stirring. The reaction mixture was stirred for 2 hours and water was removed as an azeotrope with cyclohexane. The resulting product was analyzed and determined to have surface crosslinking.

Example 29. Surface Crosslinking by Calcium Ions

10.0 g of encapsulated particles from Example 22 were added to 100 ml round bottom flask equipped with a magnetic stir bar, Dean Stark apparatus, and reflux condenser. 1.8 g of calcium chloride dissolved in 20 ml of $H_2O$ were then added with vigorous stirring. The reaction mixture was stirred for 2 hours. 20 ml of cyclohexane were added and the water was removed as an azeotrope with cyclohexane. The resulting product was analyzed and determined to have surface crosslinking.

Example 30. Surface Crosslinking by Magnesium Ions

10.0 g of encapsulated particles from Example 22 and 30 ml of cyclohexane were added to a 100 ml round bottom flask equipped with a magnetic stir bar, Dean Stark apparatus, and reflux condenser. 3.6 g of magnesium chloride dissolved in 5 ml of $H_2O$ were then added with vigorous stirring. The reaction mixture was stirred for 2 hours and water was removed as an azeotrope with cyclohexane. The resulting product was analyzed and determined to have surface crosslinking.

Example 31

Twelve grams of Example 21 (silica coated with poly sodium acrylate:acrylic acid 75:25), 25 ml of methanol, 40.5 mg of ethyleneglycol diglycidyl ether, and 1.5 g of distilled water were placed in a 100 ml flask and heated to reflux for seven hours. The reaction mixture was poured into a crystallizing dish and dried under a heat lamp for 2 hours then in a 70°C oven for 1 hour. Example 31 is a white granular material weighing 11.6 g.

Example 32

In the following procedures, particle attrition was measured under selected conditions of mechanical agitation.
Absorbent particles of the present invention having a silica based core and a polyacrylate SAP coating wherein core and SAP are present in a 1:1 weight ratio were first presieved to obtain particles in a selected particle size range of 250 to 520 microns. After subjecting the particles to mechanical agitation, analysis for the presence of fractured or separated polymer present as particles less than 74 microns in size was conducted as listed below:

| Method of Agitation | Weight % less than 74 microns |
|---|---|
| Shaker | 0.0 |
| Rolling with Small Pellets | 0.2 |
| Rolling with Large Pellets | 0.1 |

The above methods of agitation were conducted as follows:

Condition 1. Paint Shaker

10 grams of super absorbing polymer were placed in a 170 ml cylindrical metal container. This container was in turn placed in a 1 gallon paint can and placed on a paint shaker for 60 minutes. At the end of this time, the material was removed and analyzed for particle size distribution using a standard set of sieves.

Condition 2. Small Burundum Rollers

10 grams of super absorbing polymer were placed in a 170 ml cylindrical metal container along with 4 cylindrical Burundum pellets of 13 mm diameter by 13 mm in length. This container was in turn placed in a 1 gallon paint can and placed on a paint roller for 30 minutes. At the end of this time, the material was removed and analyzed for particle size distributing using a standard set of sieves.

Condition 3. Large Burundum Rollers

10 grams of super absorbing polymer were placed in a 170 ml cylindrical metal container along with 2 cylindrical Burundum pellets of 21 mm diameter by 21 mm in length. This container was in turn placed in a 1 gallon paint can and placed on a paint roller for 30 minutes. At the end of this time, the material was removed and analyzed for particle size distributing using a standard set of sieves. Based on the above results, the absorbent particles of the present invention exhibit fracture-resistant qualities based, in part, on the "weight % less then 74 microns" results. This shows highly acceptable levels of adhesion of the polymer to the solid core.

Example 33. Particle Size Control

To illustrate the predictable and controllable absorbent particle size achievable within the teachings of the present invention, the following embodiments are provided.
Absorbent particles were prepared according to Example 24, wherein silica particles prescreened to a particle size range of about 250 to 300 microns were coated with a polyacrylic acid--sodium polyacrylate at a 1:1 weight ratio of silica to polymer.

These conditions were repeated with absorbent particles prepared according to Example 25 with a silica core particle having a particle size range of about 75 microns to 150 microns.

Both granular products were lightly ground in a Braun CA Model grinder and then subjected to a series of sieves to separate the particles according to size. Data for this procedure is shown in Table VIII.

Table VIII

| Sieve | | Weight on screen g | Weight thru screen g | % Fiber Than By Weight | Median Parti-cle Size ^m | Coating Thick-ness T, ^m |
|---|---|---|---|---|---|---|
| Mesh Size | Particle Size ^m | | | | | |
| Example 24 | | | | | | |
| 40 | 420 | 22.7 | 22.3 | 50 | 240 | 60-85 |
| 60 | 250 | 15.9 | 6.4 | 14 | | |
| 100 | 150 | 3.95 | 2.4 | 5 | | |
| 200 | 75 | 1.6 | 0.8 | 1.7 | | |
| Example 25 | | | | | | |
| 40 | 420 | 4.2 | 40.8 | 90.6 | 310 | 80-117 |
| 60 | 250 | 27.7 | 13.1 | 29.1 | | |
| 100 | 150 | 8.2 | 4.9 | 10.9 | | |
| 200 | 75 | 3.5 | 1.4 | 3.1 | | |

Other embodiments of the present invention will be apparent to those skilled in the art from consideration of the specification and practice of the present invention disclosed herein. It is intended that the specification and examples be considered as exemplary only, with a true scope and spirit of the present invention being indicated by the following claims.

**Claims**

1. Use of absorbent particles for the preparation of an adsorbent device characterized in that the adsorbent particles comprise a non-colloidal water resistant solid core substantially encapsulated by a hydrogel forming polymer.

2. Use of absorbent particle according to claim 1, wherein the solid core is a hollow, mineral sphere.

3. Use of absorbent particle according to claim 1, wherein the non-colloidal water resistant solid core is an inorganic mineral.

4. Use of absorbent particles according to claim 3, wherein the inorganic mineral is selected from the group consisting of silicon dioxide, titanium dioxide, magnesium oxide, antimony oxide, clay, talc, wollastonite, synthetic amorphous silica, and calcium carbonate.

5. Use of absorbent particles according to claims 1-4, wherein the solid core is a particle with a size of from about 10 microns to about 1500 microns.

6. Use of absorbent particles according to claims 1-5, wherein the weight ratio of the solid core to hydrogel forming polymer is from about 1:4 to about 9:1.

7. Use of absorbent particles according to claims 1-6, wherein the average particle size is in the range of from about 30 microns to about 2000 microns.

8. Use of absorbent particles according to claim 1, wherein the solid core is a cereal, starch, cellulose, or gelatin in particulate form.

9. Use of absorbent particles according to claims 1-8, wherein at least two adsorbent particles are attached through interparticle surface bonding into aggregates with an average particle size of from about 30 to about 1500 microns.

10. Use of absorbent particles according to claims 1-9, wherein said hydrogel forming polymer is selected from the group consisting of polyacrylic acid, polymethacrylic acid, polymaleic acid and copolymers thereof, and their alkali metal salts; polyvinyl alcohol, polyvinyl pyrrolidone, polyvinyl alkylether, polyethylene oxide, polyacrylamide and copolymers thereof; saponified starch graft copolymers of acrylonitrile, acrylate esters, vinyl acetate; starch graft copolymers of acrylic acid, methacrylic acid, and maleic acid; and copolymers of maleic anhydride and alkyl vinyl ethers.

11. Use of absorbent particles according to claims 1-10, wherein the hydrogel polymer is cross-linked with a polyfunctional epoxy, haloepoxy, aldehyde, amine, imine, or isocyanate compound or by a polyfunctional ethylenically unsaturated compound.

12. Use of absorbent particles according to claims 1-11, wherein said hydrogel forming polymer has an outer surface that is chemically crosslinked.

13. Use of absorbent particles according to claim 10, wherein said hydrogel forming polymer has an outer surface forming a shell and an interior region and wherein said shell has a higher crosslink density than said interior region.

14. Use of absorbent particles according to claims 1-13, wherein the coating thickness of said hydrogel forming polymer is from about 2 microns to about 3000 microns.

15. Use of absorbent particles according to claim 14, wherein said coating thickness is from about 100 microns to about 300 microns.

16. Use of absorbent particles according to claim 1-15, wherein said hydrogel forming polymer has a number average molecular weight of at least about 250.000.

17. Use of absorbent particles according to claims 1, 3-16, wherein the adsorbent particles comprise a discrete particle of silica substantially encapsulated by a hydrogel forming polymer wherein said polymer comprises a partially neutralized, crosslinked polyacrylic acid; and wherein the weight ratio of silica to polymer is in the range of from about 1.0:1.5 to about 1.5:1.0.

18. Use of absorbent particles according to claim 17, wherein said particle of silica has a particle size of from about 50 microns to about 1000 microns.

19. Use of absorbent particles according to claim 18, wherein said particle size is from about 75 microns to about 500 microns.

20. Use of absorbent particles according to claims 17-19, wherein said hydrogel forming polymer has a thickness of from about 40 microns to about 150 microns.

21. Absorbent particles according to claims 1-20.

22. An absorbent device comprising hydrophilic fibers and absorbent particles according to claims 1-20, the absorbent particles being dispersed among the fibers or positioned between two layers of fibers, said fibers being preferably hydrophilic.

23. An absorbent device according to claim 22, further comprising superabsorbent particles consisting of hydrogel forming polymer.

24. An absorbent device according to claim 23, wherein said superabsorbent particles consisting of hydrogel forming polymer and said particles of the absorbent composition of claim 1 are present in a weight ratio of from about 1:9 to about 9:1.

25. An absorbent device according to claims 22-24, in which said fibers and said particles are present in a weight ratio of from about 95:5 to about 2:3.

26. An absorbent device according to claims 22-25, wherein said fibers comprise cellulose pulp and the fibers are in the form of a web, optionally collected on a forming screen.

27. An absorbent device according to claim 26, comprising:

   (a) a fibrous web of bonded or unbonded hydrophilic fibers; and
   (b) a plurality of absorbent particles of claim 21 interspersed within said web,

   wherein said solid core is an inorganic mineral in particulate form having a particle size in the range of from about 10 microns to about 1550 microns, and wherein said absorbent particles comprise 20-70% by weight of said solid core.

28. An absorbent device according to claims 22-27, being a disposable diaper, a female sanitary napkin, a urinary incontinence pad, a bed pad, a pad for absorbing fluids from food, a bandage.

29. An absorbent article comprising a fluid-permeable topsheet material adjacent at least one layer of the absorbent device according to claims 22-28, and a liquid-impermeable backing material adjacent said absorbent device and opposite said fluid-permeable topsheet material.

30. An absorbent article according to claim 29, comprising at least one volumetric zone comprising hydrophilic fibers and particles comprising non-colloidal water resistant solid cores substantially encapsulated by a hydrogel forming polymer.

31. An absorbent article according to claim 30, in which said volumetric zone or zones occupies at least about 2% of the volume of said device in the dry state.

32. Process for preparing the absorbent particles of claim 21, comprising the steps of:

   (a) individually suspending non-colloidal water resistant solid particles in a water immiscible solvent in the presence of a surface active agent;
   (b) suspending in said water immiscible solvent an aqueous solution of an ethylenically unsaturated monomer capable of polymerization into a hydrogel forming polymer and an initiator;
   (c) polymerizing said monomer such that said non-colloidal water resistant solid particles are individually and substantially encapsulated by said hydrogel forming polymer to form absorbent particles; and
   (d) separating and drying said absorbent particles.

33. Process for preparing the absorbent particles according to claim 32, wherein said polymer is cross-linked with polyfunctional cross-linking agents which are included in the aqueous solution in step (b), or at the end of step (c) and prior to step (d), or after step (d) by suspending said absorbent particles in a solvent containing a polyfunctional cross-linking agent.

34. Process for preparing the absorbent particles according to claims 32-33, wherein the diameter of the absorbent particles $D_{AP}$ ranges from about 30 to about 2000 microns, the diameter of the solid core $D_{CP}$ ranges from about 10 to about 150 microns, the thickness T of the hydrogel polymer coating ranges from about 10 to about 995 microns.

35. Process for preparing the absorbent particles according to claims 32-34, wherein said surface active agent has an HLB factor of from about 2 to about 12, preferably 3 -5.

36. Process for preparing the absorbent particles according to claim 35, wherein said surface active agent is a fatty ester of a sugar optionally reacted with ethylene oxide, the solvent being cyclohexane, the unsaturated monomer being acrylic acid, optionally partially neutralized acrylate-acrylic acid mixture.

37. Process for preparing the absorbent article according to claims 29-31, comprising:

   (a) dispersing absorbent particles of claim 21 into an absorbent core comprising fibers;
   (b) interposing said absorbent core between a liquid pervious topsheet and a liquid impervious backsheet such that an outer edge of said topsheet and said backsheet are in contact with each other; and
   (c) sealing said edges together to create a liquid barrier containing said absorbent core.

FIG. 1

FIG. 2

FIG. 3

*FIG. 4*

*FIG. 7*

FIG. 5

FIG. 6

**European Patent Office**

## EUROPEAN SEARCH REPORT

Application Number

EP 95 11 1658

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | US-A-4 587 308 (M. MAKITA)<br>* column 2, line 23 - column 5, line 25; claims; examples * | 1-37 | A61L15/60 |
| X | EP-A-0 537 053 (SOCIÉTÉ FRANCAISE HOECHST) | 1-8, 10-22 | |
| Y | * page 2, line 36 - line 38 *<br>* page 2, line 46 - page 3, line 39 *<br>* claims * | 9,32-37 | |
| X | EP-A-0 457 660 (SOCIÉTÉ FRANCAISE HOECHST)<br><br>* page 2, line 1-6 *<br>* page 2, line 19 - line 21 *<br>* page 2, line 29 - page 3, line 21 *<br>* claims * | 1-8,21, 22,32-37 | |
| X | PATENT ABSTRACTS OF JAPAN<br>vol. 12, no. 501 (C-556) 27 December 1988<br>& JP-A-63 210 109 (KANAE KAGAKU KOGYO KK)<br>31 August 1988<br>* abstract * | 1-37 | |
| Y | WO-A-91 15177 (PROCTER & GAMBLE)<br>* page 48, paragraph 2 *<br>* page 52, paragraph 2 - page 53, paragraph 2 *<br>* claims * | 9,32-37 | **TECHNICAL FIELDS SEARCHED** (Int.Cl.6)<br><br>A61L |
| D | & US-A-5 180 622 | | |
| Y | WO-A-93 24153 (DOW CHEMICAL)<br>* page 4, line 27 - page 6, line 6; examples * | 32-37 | |
| A | EP-A-0 420 248 (KIMBERLY-CLARK CORP.)<br>* page 5, line 5 - line 21 *<br>* page 7, line 40 - line 44 * | 1-37 | |
| | -/-- | | |

The present search report has been drawn up for all claims

| Place of search | Date of completion of the search | Examiner |
|---|---|---|
| THE HAGUE | 7 December 1995 | Cousins-Van Steen, G |

EPO FORM 1503 03.82 (P04C01)

European Patent Office

**EUROPEAN SEARCH REPORT**

Application Number

EP 95 11 1658

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 735 987 (Y. MORITA) | | |
| A | WO-A-90 08789 (DOW CHEMICAL) | | |
| A | DE-A-36 37 057 (LION CORPORATION) | | |
| A,D | US-A-3 932 322 (D. DUCHANE) | | |
| A | EP-A-0 339 461 (KIMBERLY-CLARK) | | |
| D | & US-A-5 147 343 | | |

TECHNICAL FIELDS
SEARCHED (Int.Cl.6)

The present search report has been drawn up for all claims

| Place of search | Date of completion of the search | Examiner |
|---|---|---|
| THE HAGUE | 7 December 1995 | Cousins-Van Steen, G |